# EUROPÄISCHE PATENTSCHRIFT

(11) **EP 2 663 346 B1**
(45) Veröffentlichungstag und Bekanntmachung des Hinweises auf die Patenterteilung: **21.06.2017**
(21) Anmeldenummer: 12701668.1
(22) Anmeldetag: 09.01.2012
(51) Int. Cl.: A61M 1/16

(54) **HERSTELLUNG VON INDIVIDUALKONZENTRAT**
PRODUCTION OF INDIVIDUALIZED CONCENTRATE
PRODUCTION DE CONCENTRÉ INDIVIDUEL

(30) Priorität: 10.01.2011 DE 102011008185; 10.01.2011 US 201161457131 P
(43) Veröffentlichungstag der Anmeldung: 20.11.2013
(73) Patentinhaber: Fresenius Medical Care Deutschland GmbH, 61352 Bad Homburg (DE)
(72) Erfinder: KLOEFFEL, Peter, 97720 Nüdlingen (DE)
(74) Vertreter: Rau, Jenspeter
(86) Internationale Anmeldenummer: PCT/EP2012/000057
(87) Internationale Veröffentlichungsnummer: WO 2012/095289

(56) Entgegenhaltungen:
- DE-A1-102007 009 269
- DE-A1-102009 031 473
- DE-B3- 10 313 965
- US-A- 5 326 476

## Beschreibung

Die Erfindung betrifft ein Verfahren zur Herstellung eines Individualkonzentrates für die Dialyse nach dem Oberbegriff des Anspruchs 1 und eine hierzu geeignete Vorrichtung gemäß Anspruch 11 zur Durchführung des Verfahrens nach Anspruch 1 gemäß Anspruch 14. Dieses Individualkonzentrat kann in seiner Zusammensetzung optimal an den speziellen Bedarf des einzelnen Patienten angepasst werden, ohne dass eine größere Auswahl von Konzentraten in der Klinik vorgehalten werden müssen. Durch ein System von Konzentraten, die von der Maschine automatisiert durchmischt werden, wird der Arbeitsaufwand für das Bedienpersonal sowie das Kontaminationsrisiko verringert.

Lösungen für die Hämodialyse werden insbesondere bei der Behandlung chronischen Nierenversagens häufig online von einer Portionierungseinheit der Dialysemaschine aus Konzentraten und hochreinem Wasser hergestellt. Der Dialysemaschine wird das hochreine Wasser meist durch eine Zentralversorgung zugeführt, während die benötigten Elektrolyte in Form von Konzentraten, abgepackt in Beuteln oder Kanistern, durch das Pflegepersonal an der Dialysemaschine bereitgestellt werden.

Bei der heute üblichen Bicarbonat-Dialyse werden bedingt durch die benötigte Elektrolytzusammensetzung der Lösung für die Hämodialyse oder ähnliche Verfahren üblicherweise zwei Konzentrate benötigt, nämlich ein basisches Konzentrat, das den Puffer Bicarbonat und gegebenenfalls Kochsalz enthält, und ein saures Konzentrat, das die übrigen Elektrolyte, wie z.B. Magnesium, Kalzium, Kalium, Glucose und eine Säure enthält. Das basische Konzentrat wird meist als Trockenkonzentrat geliefert, kann aber auch in Form von Flüssigkonzentrate bereitgestellt werden. Das saure Konzentrat ist meist ein Flüssigkonzentrat. Flüssigkonzentrate werden üblicherweise in Kunststoffkanistern mit einem Volumen von 3-10 l Kanistern geliefert. Die beiden Konzentrate werden durch die Konzentratpumpen der Portionierungseinheit der Dialysemaschine angesaugt und in der Maschine in einem definierten Verhältnis mit Wasser verdünnt, um die gewünschte Elektrolytkonzentration zu erhalten. Die so erhaltene Elektrolytlösung entspricht weitgehend der Zusammensetzung von physiologischem, also gesundem Blutplasma und kann dann zur Behandlung des chronischen Nierenversagens durch Hämodialyse verwendet werden.

Das Versagen der Nieren kann von einer Vielzahl von Krankheiten ausgelöst werden. Weiterhin stellt die Dialysebehandlung selbst einen schweren Eingriff in den menschlichen Organismus dar, der unterschiedliche Komplikationen und Comorbiditäten verursachen kann. Deshalb kann sich die Ausgangssituation zu Beginn der Behandlung von Patient zu Patient stark unterscheiden. So werden bei manchen Patienten sehr hohe Kaliumkonzentrationen vor der Dialyse festgestellt. Um die Dialyse schonend zu gestalten, kann hier die Kaliumkonzentration auch im Dialysat etwas höher gewählt werden. Gleiches ist auch für die anderen Elektrolyte denkbar. Während man bei den meisten Dialysepatienten eine zu hohe Phosphatbelastung feststellt, leiden manche Patienten, die sehr intensiv dialysiert wurden, unter einer Hypophosphatämie, die durch Phosphat in der Dialyselösung gelindert werden kann. Eine weitere Herausforderung, mit der Dialysepatienten oft zu kämpfen haben, ist die Mangelernährung. Hier kann durch Zusatz von Glucose oder Aminosäuren zum Konzentrat eine Verbesserung erreicht werden. Weiterhin kann der unbeabsichtigten Entfernung von Spurenelementen aus dem Blut durch die Dialysebehandlung bei Bedarf durch Substitution dieser in der Dialyselösung entgegengewirkt werden. Die individuellen Anforderungen an die genaue Zusammensetzung der verwendeten Dialyselösung zeigen also ein großes Spektrum, und die individuelle Anpassung der Dialyselösung an die jeweiligen Bedürfnisse des Patienten kann Nebenwirkungen minimieren und den Behandlungserfolg verbessern.

Dem gegenüber steht die räumlich begrenzte Lagerkapazität in der Klinik, so dass immer nur eine beschränkte Anzahl unterschiedlicher Dialysekonzentrate vorgehalten werden kann. Gleichzeitig würde die Lagerhaltung und Herstellung sehr vieler unterschiedlicher Konzentrate eine aufwendige Logistik erfordern.

In der DE 3224823 wird dieses Problem dadurch gelöst, dass man das Konzentrat für die Lösung für die Hämodialyse modulartig zusammensetzt. Zu einer Stammlösung, deren Elektrolytzusammensetzung nach Verdünnung durch die Portioniereinheit der Dialysemaschine in etwa der physiologischen Elektrolytzusammensetzung des Blutes entspricht, werden ein oder mehrere Spezialkonzentrate als Zusatzlösung zugegeben, die die gewünschte Zusatzkomponente enthalten. Die Stammlösung wird in einer Menge von neun Litern in einem Kanister vorgelegt. Die Zusatzlösungen bzw. Spezialkonzentrate werden in Mengen von 10-500 ml zugegeben. Die Spezialkonzentrate enthalten jeweils nur eine Komponente, die entweder in Stammlösung nicht enthalten sind, oder deren Konzentration im Vergleich zur Stammlösung erhöht werden soll.

In der WO 99/07419 wird die Zugabe von Vitaminen und Spurenelementen zum flüssigen Bicarbonat-Konzentrat beschrieben.

Das Konzentrat für die Herstellung der Lösung für die Hämodialyse muss allerdings homogen sein, sonst ist eine sichere Behandlung nicht gewährleistet. In der heutigen Praxis wird deshalb nach Zusage des Spezialkonzentrates zum sauren oder basischen Konzentrat für die Bicarbonatdialyse die resultierend Lösung durch intensives Schütteln des Kanisters durch das Pflegepersonal gemischt. Dies bedeutet eine körperliche Belastung für den Anwender. Der Kanister muss für die Zugabe des Spezialkonzentrates geöffnet werden. Vor Schütteln des Kanisters wird dieser wieder mit dem Deckel verschlossen. Bei diesen Arbeitsschritten kann es zu einer Kontamination des Konzentrates kommen.

Der Erfindung liegt die Aufgabe zugrunde, ein für den jeweiligen Patienten speziell angepasstes Individualkonzentrat aus mindestens einem ersten und einem zweiten Konzentrat herzustellen, wobei der Arbeitsaufwand für das Personal und das Kontaminationsrisiko minimiert und gleichzeitig eine vollständige Durchmischung gewährleistet werden. Eine weitere Aufgabe der Erfindung liegt in der Bereitstellung einer Vorrichtung für die Herstellung eines Individualkonzentrates.

Nach der Lehre der Erfindung werden diese Aufgaben durch ein Verfahren zur Herstellung eines flüssigen Individualkonzentrates für die Dialyse gemäß Anspruch 1 und durch eine Vorrichtung gemäß Anspruch 11 Vorteilhafte Ausgestaltungen sind Gegenstand der Unteransprüche.

Nach der Lehre der Erfindung wird diese Aufgabe durch ein Verfahren zur Herstellung eines in einem Behältnis befindlichen, flüssigen Individualkonzentrates für die Dialyse aus einem ersten Konzentrat und mindestens einem zweiten Konzentrat mittels einer Vorrichtung, umfassend eine Dialysemaschine mit mindestens einer Druckluftquelle, mindestens einer Druckluftleitung zwischen Konzentratleitung und Druckluftquelle und mindestens einem Konzentratansaugstab, der an die Konzentratleitung angeschlossen ist, gelöst, wobei die Durchmischung der beiden Konzentrate durch Druckluft erfolgt, die von der in der Dialysemaschine befindlichen Druckluftquelle über die Druckluftleitung, Konzentratleitung und angeschlossenem Konzentratansaugstab in das Behältnis eingebracht wird.

Vor Beginn der Behandlung wird die Zusammensetzung des Individualkonzentrates für den speziellen Patienten und das dafür benötigte erste Konzentrat und das zweite Konzentrat, sowie gegebenenfalls weitere Konzentrate, festgelegt.

Von den in dem Verfahren eingesetzten Konzentraten ist das 1. Konzentrat vorzugsweise ein Standardkonzentrat. Bei der Bicarbonatdialyse werden zwei Standardkonzentrate, ein saures und ein basisches Konzentrat, benötigt. Sowohl das saure als auch das basische Konzentrat können verwendet werden, um das erfindungsgemäße Verfahren durchzuführen. Zur Durchführung des Verfahrens können handelsübliche Flüssigkonzentrate, z.B. AC-F Liquid 1+44 oder BC-F-Liquid Bicarbonate concentrate 8,4% von Fresenius Medical Care, für die Hämodialyse verwendet werden. Das Standardkonzentrat wird bevorzugt in einer Menge von 3-10 l in einem Kunststoffkanister zur Verfügung gestellt, kann aber auch in einem Beutel oder einem anderen geeigneten Behältnis geliefert werden.

Bei dem zweiten und eventuell weiteren Konzentraten handelt es sich vorzugsweise um Spezialkonzentrate, die in einer Menge von 5-200 ml, vorzugsweise von 10-50 ml zur Verfügung gestellt werden. Die Konzentrationsverhältnisse im Standardkonzentrat sollen sich durch die Zugabe des Individualkonzentrates für die übrigen Komponenten nicht ändern. Deshalb werden die Individualkonzentrate immer in einer möglichst kleinen Lösungsmenge zugegeben. Anders als im Stand der Technik muss dann keine Volumenanpassung erfolgen.

Die Spezialkonzentrate enthalten bevorzugt pro Verpackungseinheit jeweils nur eine Komponente, wie z.B. Kalium, Natrium, Calcium, Magnesium oder Phosphat, Glucose, Vitamine, Aminosäuren oder Spurenelemente. Es kann jede Substanz verwendet werden, die mit den Komponenten des Standardkonzentrates kompatibel ist, und die dem Patienten entweder zugeführt werden soll oder deren Entfernung aus dem Blut durch die Dialyse verhindert werden soll. Die Verpackungseinheit kann ein Beutel, eine spezielle Kartusche oder eine in eine Spritze aufgezogene Lösung sein.

Das Spezialkonzentrat kann z.B. in das Konzentratbehältnis des Standardkonzentrates gegeben werden, da dieses nie vollständig gefüllt ist. Wird das eher geringe Volumen des Spezialkonzentrates zu dem deutlich höheren Volumen des Standardkonzentrates gegeben, erhält man zunächst eine eher inhomogene Mischung, die durch das erfindungsgemäße Verfahren homogenisiert wird.

Vor Beginn der Dialyse wird standardmäßig der Konzentratansaugstab, der mit der Konzentratleitung der Maschine verbunden ist, in das Konzentratbehältnis eingebracht, das entweder reines Standardkonzentrat oder Standardkonzentrat und Spezialkonzentrat enthält. Der Konzentratansaugstab dient während der Behandlung dazu, das Konzentrat mithilfe einer Konzentratpumpe durch eine Konzentratleitung der Portioniereinheit der Dialysemaschine zuzuführen.

Bei dem erfindungsgemäßen Verfahren wird nun über diesen Konzentratansaugstab Luft in die Mischung der Konzentrate eingeleitet, und diese so zu einem Individualkonzentrat homogenisiert. Die Luft wird dabei von einer in der Dialysemaschine befindlichen Druckluftquelle, wie z.B. einem Kompressor, geliefert. Solche Druckluftquellen sind in Dialysemaschinen oft bereits vorgesehen und werden z.B. zur Durchführung von Drucktests oder zum Setzen von Pegeln in Luftabscheidekammern verwendet. Es kann allerdings auch eine speziell nur für diesen Zweck vorgesehene Druckluftquelle verwendet werden.

Die Druckluftquelle liefert die Druckluft in eine Druckluftleitung. Nach einer Ausführungsform des erfindungsgemäßen Verfahrens wird dann ein Ventil in der Druckluftleitung geöffnet, und die Druckluft gelangt aus der Druckluftleitung in die Konzentratleitung. Die Druckluftleitung wird dabei bevorzugt zwischen Konzentratpumpe und Konzentratansaugstab mit der Konzentratleitung verbunden. Von dort wird die Druckluft über den Konzentratansaugstab in das Konzentratbehältnis mit der inhomogenen Mischung der Konzentrate geführt. Das äußere Ende des Konzentratansaugstabes befindet sich in Bodennähe des Kanisters, um eine möglichst vollständige Entleerung während der Konzentratentnahme zu ermöglichen. Die Luft, die aus dem Konzentratansaugstab tritt, steigt in Form von Luftblasen von unten an die Oberfläche. Durch diese Blasen werden die Konzentrate homogenisiert, d.h. vollständig durchmischt. Die Druckluft wird in einer Ausführungsform des erfindungsgemäßen Verfahrens über einen Zeitraum von 5-60 s in einer Menge von 1-101 Luft pro Minute in die beiden Konzentrate eingeblasen. In einem besonders bevorzugten Verfahren wird die Druckluft zur Minimierung eines Kontaminationsrisikos vor Einblasen in die Mischung der Konzentrate mithilfe eines Sterilfilters, der gegebenenfalls in die Druckluftleitung eingebaut ist, sterilfiltriert.

Der Anwender kann die Durchführung des Verfahrens durch eine Eingabe im Bedienfeld der Dialysemaschine initiieren, das mit einer Steuereinheit verbunden ist. Die Eingabe kann auf unterschiedliche Arten erfolgen. Das Bedienfeld kann z.B. ein Touch-Screen sein, an dem die unterschiedlichen Behandlungsoptionen vom Bediener gewählt werden können. Auf diesem Touchscreen kann sich ein Button "Luft" befinden, der bei Betätigung der Steuereinheit signalisiert, dass das erfindungsgemäße Verfahren durchgeführt werden soll. Das Signal kann aber auch von einer mechanischen Taste an dem Dialysegerät, die bei Bedarf vom Bediener betätigt wird, erfolgen. Die vorgenannten Verfahrensschritte werden dann von einer Steuereinheit, die konfiguriert ist das erfindungsgemäße Verfahren durchzuführen, veranlasst und gegebenenfalls überwacht.

Zur Durchführung des erfindungsgemäßen Verfahrens kann eine Vorrichtung zur Durchführung einer Dialysebehandlung mit einer Portionierungseinheit zur online Bereitstellung von Lösungen für die Hämodialyse mit mindestens einer Konzentratleitung und daran angeschlossenem Konzentratansaugstab und mindestens einer Druckluftquelle verwendet werden, wobei diese Portionierungseinheit eine Druckluftleitung von der Druckluftquelle zu der an den Konzentratansaugstab angeschlossenen Konzentratleitung aufweist. Bei dieser Druckluftquelle kann jeder handelsübliche Kompressor verwendet werden, der in der Lage, ist einen Fluss von 1-10 1 Luft pro Minute zu liefern.

In einer bevorzugten Ausführungsform weist die Druckluftleitung weiterhin einen Sterilfilter auf, damit die Luft, die durch das Konzentrat geleitet wird, dieses nicht kontaminiert. Der Sterilfilter dient zur Sterilisierung der zur Durchmischung der Konzentrate verwendeten Luft. Bei dem Sterilfilter kann es sich z.B. um einen handelsüblichen Membranfilter handeln, es ist allerdings auch jeder andere Filter, der zur Sterilfiltrierung von Luft geeignet ist, verwendbar.

In einer weiteren bevorzugten Ausführungsform ist am Verbindungspunkt von Druckluftleitung und Konzentratleitung ein Ventil vorgesehen. In einer bevorzugten Ausführungsform handelt es sich um ein Rückschlagventil. In einer besonders bevorzugten Ausführungsform handelt es sich um ein elektromagnetisches Absperrventil. Es kann jedoch auch jedes andere Ventil, das zur Abtrennung der Druckluftleitung von der Konzentratleitung geeignet ist, verwendet werden. Das Ende der Konzentratleitung, das aus der Dialysemaschine herausführt, ist mit einem Konzentratansaugstab verbunden. Dieser Konzentratansaugstab wird in das Konzentratbehältnis eingetaucht. Um eine möglichst vollständige Entleerung des Konzentratbehältnisses, z.B. eine Kunststoffkanister, zu ermöglichen, befindet sich das äußere Ende des Konzentratansaugstabes am Boden des Behältnisses. Aus dem Ende des Konzentratansaugstabes wird die Luft aus der Druckluftquelle in den Kanister mit dem Standardkonzentrat und dem Spezialkonzentrat geleitet, wodurch diese zu einem Individualkonzentrat durchmischt werden.

Weitere Einzelheiten und Vorteile der Erfindung werden anhand eines in der einzigen Zeichnung dargestellten Ausführungsbeispiels näher beschrieben. Es zeigt:

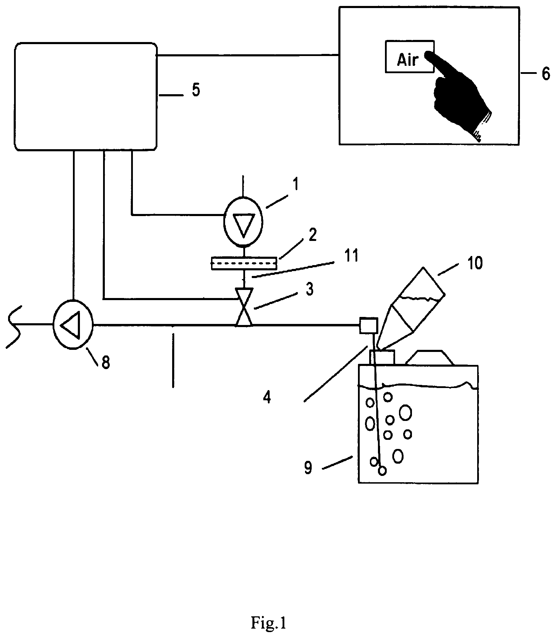

Figur 1: Vorrichtung zur automatischen Durchmischung von Konzentraten

Wie im Stand der Technik beschrieben werden nach Festlegung der Zusammensetzung des Individualkonzentrates durch den behandelnden Arzt zu dem ersten Konzentrat, z.B. dem Standardkonzentrat 9 das benötigte zweite Konzentrat, z.B. ein Spezialkonzentrat 10 gegeben. Als erstes Konzentrat bzw. Standardkonzentrat 9 wird hier z.B. ein 4,7 l Kanister (1+44) AC-F 119/5 von Fresenius Medical Care verwendet. Die aus diesem Konzentrat nach Verdünnung erhaltene Lösung für die Hämodialyse hat eine Kaliumchloridkonzentration von 1mmol/l. Diese kann auf 2 mmol/l erhöht werden, indem man 50 ml eines Spezialkonzentrates 9 mit einer KCl-Konzentration von 4,2 mol/l zu dem Standardkonzentrat 10 zudosiert.

In die so erhaltene, nicht homogene Mischung der Konzentrate wird der Konzentratansaugstab 4 eingebracht. Da das Konzentrat möglichst vollständig aus dem Behältnis entfernt werden soll, befindet sich das Ende des Konzentratansaugstabes im unteren Bereich des Behältnisses. Der Bediener signalisiert nun der Steuereinheit 5 der Dialysemaschine durch Eingabe in einem Bedienfeld 6, dass das Verfahren zur Durchmischung der Konzentrate durchgeführt werden soll. Die Steuereinheit 5 veranlasst nun, dass die Druckluftquelle 1 Druckluft in einer Menge von z.B. 2 l Luft pro Minute in die Druckluftleitung 11 fördert. Gleichzeitig wird das elektromagnetische Absperrventil 3 geöffnet. Nachdem die Druckluft in der Druckluftleitung 11 durch den Sterilfilter 2 sterilfiltriert wurde, gelangt sie durch das geöffnete magnetische Absperrventil 3 in die Konzentratleitung 7. Die Konzentratpumpe 8 steht zu diesem Zeitpunkt und schließt die Konzentratleitung 7 in Richtung der Portioniereinheit ab. Die Druckluft wird dann über den Konzentratansaugstab in das Standardkonzentrat 9, dem das Spezialkonzentrat 10 zugegeben wurde, gefördert und homogenisiert diese Mischung auf diese Weise innerhalb von z.B. 30 Sekunden zu einem Individualkonzentrat.

Das erfindungsgemäße Verfahren ermöglicht ein individuelle Anpassung der verwendeten Dialyselösung an die Bedürfnisse des einzelnen Patienten, ohne dass eine große Anzahl unterschiedlicher Konzentrate vorgehalten werden muss. Für das Pflegepersonal ist der zusätzliche Aufwand minimal

## Patentansprüche

1. Verfahren zur Herstellung eines in einem Behältnis befindlichen, flüssigen Individuallconzentrates für die Dialyse aus einem ersten, flüssigen Konzentrat und mindestens einem zweiten, flüssigen Konzentrat mittels einer Vorrichtung umfassend eine Dialysemaschine mit mindestens einer Druckluftquelle, mindestens einer Konzentratleitung, mindestens einer Druckluftleitung zwischen Konzentratleitung und Druckluftquelle und mindestens einem Konzentratansaugstab, der an die Konzentratleitung angeschlossen ist, **gekennzeichnet dadurch, dass** die Durchmischung der Konzentrate mittels Druckluft erfolgt, die von einer in der Dialysemaschine befindlichen Druckluftquelle über die Druckluftleitung, Konzentratleitung und angeschlossenem Konzentratansaugstab in das Behältnis eingebracht wird.

2. Verfahren nach Anspruch l **gekennzeichnet dadurch, dass** das erste Konzentrat ein Standardkonzentrat für die Bicarbonat-Dialyse ist.

3. Verfahren nach Anspruch 2 **gekennzeichnet dadurch, dass** das Standardkonzentrat ein saures Konzentrat oder ein basisches Konzentrat für die Bicarbonat-Dialyse sein kann.

4. Verfahren nach Anspruch 2 oder 3 **gekennzeichnet dadurch, dass** das Standardkonzentrat in einer Menge von 3-101 zur Verfügung gestellt wird.

5. Verfahren nach Anspruch nach einem der vorhergehenden Ansprüche **gekennzeichnet dadurch, dass** das zweite und die gegebenenfalls weiteren Konzentrate Spezialkonzentrate sind.

6. Verfahren nach Anspruch 5 **gekennzeichnet dadurch, dass** die Spezialkonzentrate in einer Menge von 5-200 ml zur Verfügung gestellt werden.

7. Verfahren nach einem der vorgehenden Ansprüche **gekennzeichnet dadurch, dass** zur Lieferung der Druckluft in die Konzentratleitung ein Ventil zwischen Druckluftleitung und Konzentratleitung geöffnet wird.

8. Verfahren nach einem der vorhergehenden Ansprüche **gekennzeichnet dadurch, dass** die Druckluft über einen Zeitraum von 5-60 s in das erste und die weiteren Konzentrate eingebracht wird.

9. Verfahren nach einem der vorhergehenden Ansprüche **gekennzeichnet dadurch, dass** die Druckluft in einer Menge von 1-101 Luft pro Minute geliefert wird.

10. Verfahren nach einem der vorhergehenden Ansprüche **gekennzeichnet dadurch, dass** alle Steuer- und Regelvorgänge zur Durchführung des Verfahrens von einer Steuereinheit veranlasst werden.

11. Vorrichtung zur Durchführung einer Dialysebehandlung mit einer Portionierungseinheit zur on-line Bereitstellung von Lösungen für die Hämodialyse mit mindestens einer Konzentratleitung und daran angeschlossenem Konzentratansaugstab und mindestens einer Druckluftquelle **gekennzeichnet dadurch, dass** diese Portionierungseinheit eine Druckluftleitung von der Druckluftquelle zu der an den Konzentratansaugstab angeschlossenen Konzentratleitung aufweist.

12. Vorrichtung nach Anspruch 11 **gekennzeichnet dadurch, dass** sich in der Druckluftleitung ein Sterilfilter befindet.

13. Vorrichtung nach Anspruch 11 oder 12 **gekennzeichnet dadurch, dass** sich zwischen der Druckluftleitung und der Konzentratleitung ein Ventil angeordnet ist.

14. Vorrichtung nach Anspruch 11 bis 13 **gekennzeichnet dadurch, dass** sie eine Steuereinheit aufweist, die alle zur Durchführung des Verfahrens nach Anspruch 1 nötigen Steuer- und Regelvorgänge veranlasst.

## Claims

1. A method for preparing in a container an individual liquid concentrate for dialysis, from a first liquid concentrate and at least one second liquid concentrate by means of a device, comprising a dialysis machine with at least one compressed air source, at least one concentrate line, at least one compressed air line between the concentrate line and the compressed air source and at least one concentrate intake rod, which is connected to the concentrate line,
**characterized in that**
the concentrates are mixed thoroughly by means of compressed air which is introduced into the container from a compressed air source situated in the dialysis machine, a concentrate line and a connected concentrate intake rod.

2. The method according to claim 1,
**characterized in that**
the first concentrate is a standard concentrate for bicarbonate dialysis.

3. The method according to claim 2,
**characterized in that**
the standard concentrate may be an acidic concentrate or a basic concentrate for bicarbonate dialysis.

4. The method according to claim 2 or 3,
**characterized in that**
the standard concentrate is supplied in an amount of 3-10 L.

5. The method according to any one of the preceding claims,
**characterized in that**
the second concentrate and the optional additional concentrates are special concentrates.

6. The method according to claim 5,
**characterized in that**
the special concentrates are supplied in an amount of 5-200 mL.

7. The method according to any one of the preceding claims,
**characterized in that**
a valve is opened between the compressed air line and the concentrate line to supply the compressed air into the concentrate line.

8. The method according to any one of the preceding claims,
**characterized in that**
the compressed air is introduced into the first concentrate as well as the additional concentrates over a period of 5-60 seconds.

9. The method according to any one of the preceding claims,
**characterized in that**
the compressed air is supplied in an amount of 1-10 L of air per minute.

10. The method according to any one of the preceding claims,
**characterized in that**
all the control and regulating processes for carrying out the method are controlled by a control unit.

11. A device for carrying out a dialysis treatment with an apportioning unit for online supply of solutions for hemodialysis with at least one concentrate line and a concentrate intake rod connected thereto and at least one compressed air source,
**characterized in that**
this apportioning unit has a compressed air line from the compressed air source to the concentrate line,
which is connected to the concentrate intake rod.

12. The device according to claim 11,
**characterized in that**
a sterile filter is provided in the compressed air line.

13. The device according to claim 11 or 12,
**characterized in that**
a valve is arranged between the compressed air line and the concentrate line.

14. The device according to claims 11 to 13,
**characterized in that**
it has a control unit, which controls all the control and regulating processes required to carry out the method according to claim 1.

## Revendications

1. Procédé de préparation d'un concentré individuel liquide se trouvant dans un récipient pour la dialyse à partir d'un premier concentré liquide et d'au moins un second concentré liquide au moyen d'un dispositif comprenant une machine de dialyse dotée d'au moins une source d'air comprimé, d'au moins une conduite de concentré, d'au moins une conduite d'air comprimé entre la conduite de concentré et la source d'air comprimé et d'au moins une barre d'aspiration de concentré qui est raccordée à la conduite de concentré, **caractérisé en ce que** le mélange des concentrés est effectué au moyen d'air comprimé qui est incorporé dans le récipient par une source d'air comprimé se trouvant dans la machine de dialyse par l'intermédiaire de la conduite d'air comprimé, de la conduite de concentré et de la barre d'aspiration de concentré raccordée.

2. Procédé selon la revendication 1, **caractérisé en ce que** le premier concentré est un concentré standard pour la dialyse au bicarbonate.

3. Procédé selon la revendication 2, **caractérisé en ce que** le concentré standard peut être un concentré acide ou un concentré basique pour la dialyse au bicarbonate.

4. Procédé selon la revendication 2 ou 3, **caractérisé en ce que** le concentré standard est mis à disposition en une quantité de 3 à 10 1.

5. Procédé selon une des revendications précédentes, **caractérisé en ce que** le premier et le cas échéant les autres concentrés sont des concentrés spéciaux.

6. Procédé selon la revendication 5 **caractérisé en ce que** les concentrés spéciaux sont mis à disposition en une quantité de 5 à 200 ml.

7. Procédé selon une des revendications précédentes, **caractérisé en ce que**, pour délivrer l'air comprimé dans la conduite de concentré, une soupape est ouverte entre la conduite d'air comprimé et la conduite de concentré.

8. Procédé selon une des revendications précédentes, **caractérisé en ce que** l'air comprimé est incorporé pendant une durée de 5 à 60 secondes dans le premier et les autres concentrés.

9. Procédé selon une des revendications précédentes, **caractérisé en ce que** l'air concentré est délivré en une quantité de 1 à 10 1 d'air par minute.

10. Procédé selon une des revendications précédentes, **caractérisé en ce que** toutes les opérations de commande et de réglage pour la réalisation du procédé sont déclenchées par une unité de commande.

11. Dispositif de réalisation d'un traitement par dialyse comportant une unité de portionnement pour la mise à disposition en ligne de solutions pour l'hémodialyse comportant au moins une conduite de concentré et une barre d'aspiration de concentré qui lui est raccordée et au moins une source d'air comprimé, **caractérisé en ce que** cette unité de portionnement présente une conduite d'air comprimé depuis la source d'air comprimé jusqu'à la conduite de concentré raccordée à la barre d'aspiration de concentré.

12. Dispositif selon la revendication 11, **caractérisé en ce qu'**un filtre stérile se trouve dans la conduite d'air comprimé.

13. Dispositif selon la revendication, **caractérisé en ce que** 11 ou 12, **caractérisé en ce qu'**une soupape est disposée entre la conduite d'air comprimé et la conduite de concentré.

14. Dispositif selon la revendication, **caractérisé en ce que** 11 à 13, **caractérisé en ce qu'**il présente une unité de commande qui déclenche toutes les opérations de commande et de réglage nécessaires à la réalisation du procédé selon la revendication 1.
